(19)

Europäisches
Patentamt
European
Patent Office
Office européen
des brevets

(11) **EP 3 333 800 A1**

(12) **EUROPEAN PATENT APPLICATION**
published in accordance with Art. 153(4) EPC

(43) Date of publication:
**13.06.2018 Bulletin 2018/24**

(21) Application number: **16832674.2**

(22) Date of filing: **06.07.2016**

(51) Int Cl.:
**G06Q 50/22** (2018.01)

(86) International application number:
**PCT/JP2016/070040**

(87) International publication number:
**WO 2017/022395 (09.02.2017 Gazette 2017/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.08.2015 JP 2015154775**

(71) Applicant: **Kaya, Takafumi**
**Ota-ku, Tokyo 1440052 (JP)**

(72) Inventor: **Kaya, Takafumi**
**Ota-ku, Tokyo 1440052 (JP)**

(74) Representative: **Regimbeau**
**20, rue de Chazelles**
**75847 Paris Cedex 17 (FR)**

(54) **HEALTH INFORMATION UTILIZATION SYSTEM, METHOD, AND PROGRAM**

(57) The purpose of the present invention is to verify a plurality of articles relating to health information, and to provide to a user only information which is highly reliable, i.e., probably correct. [Solution] A plurality of articles relating to health information, a plurality of questions relating to each of the articles, and statistical data of results of responses to the questions are stored in a database 201 of a web server 200. Before providing the articles which are stored in the database 201 to a user, the web server 200 presents the user with the plurality of questions relating to the articles, acquires each response result to the plurality of presented questions from the user, and when each response result is acquired, reads out the statistical data associated with each response result from the database 201 and provides same to the user. An administrator terminal 300 updates the statistical data on the basis of the response results which are acquired by the web server 200.

FIG. 1

EP 3 333 800 A1

**Description**

Technical Field

The present invention relates to a health information utilization system, a method, and a program, and more particularly, to a health information utilization system, a method, and a program, which utilize information necessary for health by improving meals and daily life.

Background Art

Conventionally, a system for providing health-related information to a user is disclosed in Patent Literature 1. According to the invention disclosed in Patent Literature 1, clinical academic research papers including a huge number of health-related information present on a communication network are taken as digital data, and main information and sample information are extracted as tag information with respect to each paper. Further, the reliability of the paper and the probability of the effect on the usefulness described in the paper are calculated based on the sample information and added to tag information as ranking information, and the ranking information is crated and stored as a list for each research subject and usefulness. Therefore, the user can retrieve information, which is extracted from the paper containing the health-related information and processed and analyzed, from the user terminal.

Citation List

Patent Literature

Patent Literature 1: JP 2008-181188 A

Summary of Invention

Technical Problem

However, in the invention disclosed in Patent Literature 1, prior to providing a clinical academic research paper containing an enormous number of health-related information to a user, a plurality of questions corresponding to the health-related information are presented to the user, and it is not configured to acquire each answer result from the user for the plurality of presented questions.

For this reason, it is impossible to verify the reliability of a vast number of health-related information, that is, whether a vast number of health-related information is correct information, or erroneous or uncertain information, and there has been a problem that the erroneous information or the uncertain information can cause the user to mislead.

In addition, even when highly reliable information that would be correct is given to the user, if the user does not make measures to change his or her lifestyle based on the information, it does not mean that the user has made useful use of health information.

Therefore, it is an object of the present invention to provide health information that is useful to the user and to urge the user to usefully use the health information.

Solution to Problem

To solve the above problem, a health information utilization system according to the present invention includes:

a providing means for providing a task relating to health information to a user;
a receiving means for receiving information indicating that the task provided by the providing means has been achieved; and
a stopping means for stopping receiving the information when the number of times of receptions of the information by the receiving means exceeds a predetermined number.

Furthermore, the health information utilization system may include a resuming means for, after the reception is stopped by the stopping means, resuming provision of a stopped task when a stop release instruction is transmitted from the user.

Furthermore, a health information utilization program according to the present invention causes a user terminal to execute:

a step of providing a task relating to health information to a user;
a step of receiving information indicating that the task provided by the providing means has been achieved; and
a step of stopping receiving the information when the number of times of receptions of the information by the receiving means exceeds a predetermined number.

Advantageous Effects of Invention

According to the present invention, prior to providing a plurality of articles on health information to the user, a plurality of questions corresponding to the articles are presented to the user, and each answer result from the user for the plurality of presented questions is acquired, so that it is possible to verify a plurality of articles on health information and provide only highly reliable information that would be correct to the user.

Brief Description of Drawings

Fig. 1 is a schematic configuration diagram of a health information utilization system according to an embodiment of the present invention.
Fig. 2 is a sequence diagram illustrating a health information utilization method in the health information utilization system of Fig. 1.
Fig. 3 is an initial screen displayed on a user terminal of Fig. 1.
Fig. 4 is a diagram illustrating an example of an article list screen displayed on the user terminal of Fig. 1.
Fig. 5 is a diagram illustrating an example of a first questionnaire screen displayed on the user terminal of Fig. 1.
Fig. 6 is a diagram illustrating an example of a second questionnaire screen displayed on the user terminal of Fig. 1.
Fig. 7 is a diagram illustrating an example of a comment screen displayed on the user terminal of Fig. 1.
Fig. 8 is a diagram illustrating an example of an effect score detailed comment screen displayed on the user terminal of Fig. 1.
Fig. 9 is a diagram illustrating an example of a my try addition screen displayed on the user terminal of Fig. 1.
Fig. 10 is a diagram illustrating an example of a my try management screen displayed on the user terminal of Fig. 1.
Fig. 11 is a diagram illustrating an example of a health level screen displayed on the user terminal of Fig. 1.

Reference Signs List

100    user terminal
101    display
200    web server
201    database
202    controller
300    administrator terminal
301    display
302    operation means
400    network

Description of Embodiments

Hereinafter, embodiments of the present invention will be described with reference to the drawings.

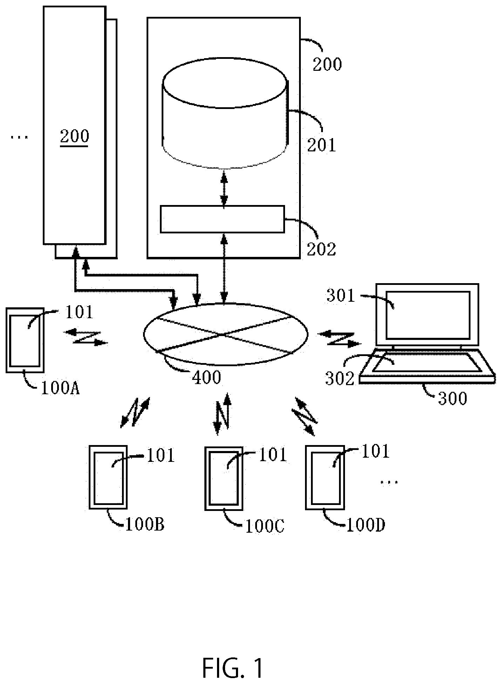

Fig. 1 is a schematic configuration diagram of a health information utilization system according to an embodiment of the present invention. The health information utilization system of Fig. 1 includes user terminals 100A to 100D, a web server 200, an administrator terminal 300, and a network 400, which will be described below.
The user terminals 100A to 100D (hereinafter, these are collectively referred to as a "user terminal 100") are configured by, for example, smartphones having a telephone function and a communication function. Therefore, a touch panel as an operation means is provided on a display 101. However, the user terminal 100 can be not only a smartphone but also various information processing devices such as a personal computer or a tablet.
In addition, although not illustrated in Fig. 1, the user terminal 100 includes a nonvolatile memory such as a flash memory which stores a health information utilization program as an application, a communication means which communicates with the web server 200 and the administrator terminal 300 via the network 400, and the like.

The web servers 200A to 200D (hereinafter, these are collectively referred to as a "web server 200") each have a database 201 and a controller 202. A plurality of articles on health information, a plurality of questions on each article, statistical data of the answer result to the question, and the like are stored in the database 201 according to an instruction from the administrator terminal 300. According to an instruction from the administrator terminal 300, the controller 202 has a means for controlling reading and writing of statistical data and the like with respect to the database 201, and a means for communicating with the user terminal 100 and the administrator terminal 300 via the network 400.

The administrator terminal 300 is configured by, for example, a notebook personal computer. Therefore, the administrator terminal 300 includes a display 301 and an operation means 302. In addition, although not illustrated in Fig. 1, the administrator terminal 300 includes a communication means for communicating with a website server related to various websites, in addition to the user terminal 100 and the web server 200 via the network 400. It should be noted that the administrator terminal 300 can be configured not only by a notebook personal computer but also by a desktop personal computer, a smartphone, a tablet, or the like.

For example, it is possible to integrate several media illustrated in Fig. 1, such as integrating the web server 200 and the administrator terminal 300, or conversely, it is possible to share some of the operations realized by the administrator terminal 300 by preparing a plurality of terminals.

The network 400 is configured by the Internet or the like, and realizes communication with the user terminal 100, the web server 200, the administrator terminal 300, and other web servers 200 in which various search sites are stored.

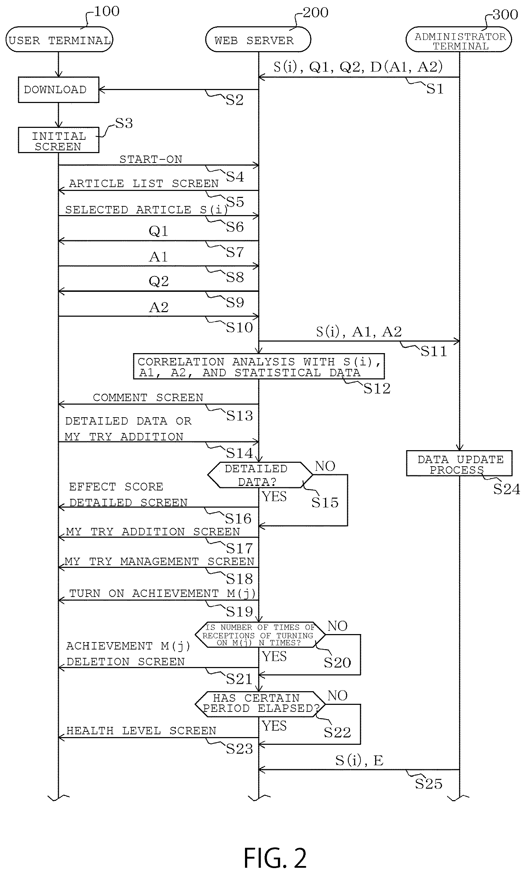

Next, a health information utilization method performed by the health information utilization system of Fig. 1 will be described with reference to Figs. 2 to 11.

Fig. 2 is a sequence diagram illustrating the health information utilization method in the health information utilization system of Fig. 1.

In Fig. 2, first, the administrator of the administrator terminal 300 inputs, to the web server 200, a plurality of articles S(i) (i = 1, 2, 3 ...) on health information, a plurality of questions on each article, for example, two questions Q1 and Q2, and statistical data D (A1; A2) of answer results A1 and A2 for the questions Q1 and Q2 (step S1).

As the plurality of articles S(i), for example, there are articles on health information relating to these subjects, such as "Pectin of an apple is effective for stomachache", "Immunity is raised to make a body that will not get sick", "Colds are prevented with nail massage for 2 minutes a day", or the like. A unique address (uniform resource locator (URL)) is assigned to each article. In the present embodiment, an operation in the case of realizing the health information utilization system by an application program installable on a smartphone or the like that is the user terminal 100 will be described as an example.

Next, the user terminal 100 downloads an application program installable on its own terminal via the network 400 and installs the application program. It should be noted that this application program may be stored in, for example, the web server 200.

Subsequently, the user terminal 100 first activates the application program in response to an instruction from a user who desires to view any one of the above-mentioned articles (step S2) .

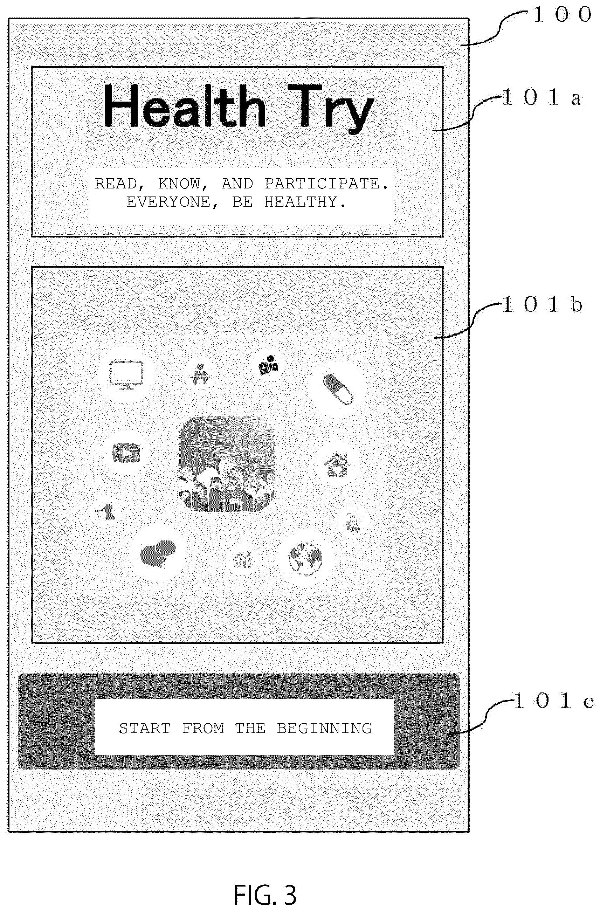

Fig. 3 is a diagram illustrating an example of an initial screen displayed on the user terminal 100. In this example, as the initial screen, a title 101a of the application program, a menu icon 101b for selecting some menus provided as a service by the present system, and a start tab 101c for starting service provision by the present system are posted.

When the user taps the start tab 101c in the initial screen displayed on the display 101 of the user terminal 100, the user terminal 100 transmits a start-on command to the web server 200 (step S4). Specifically, when the start tab 101c is tapped by the user, a URL assigned to a page of an article theme list screen is transmitted from the user terminal 100 to the web server 200.

When the start-on command is received from the user terminal 100, the web server 200 reads data of the article theme list screen from the database 201 by the controller 202 and transmits the article theme list screen to the user terminal 100 (step S5).

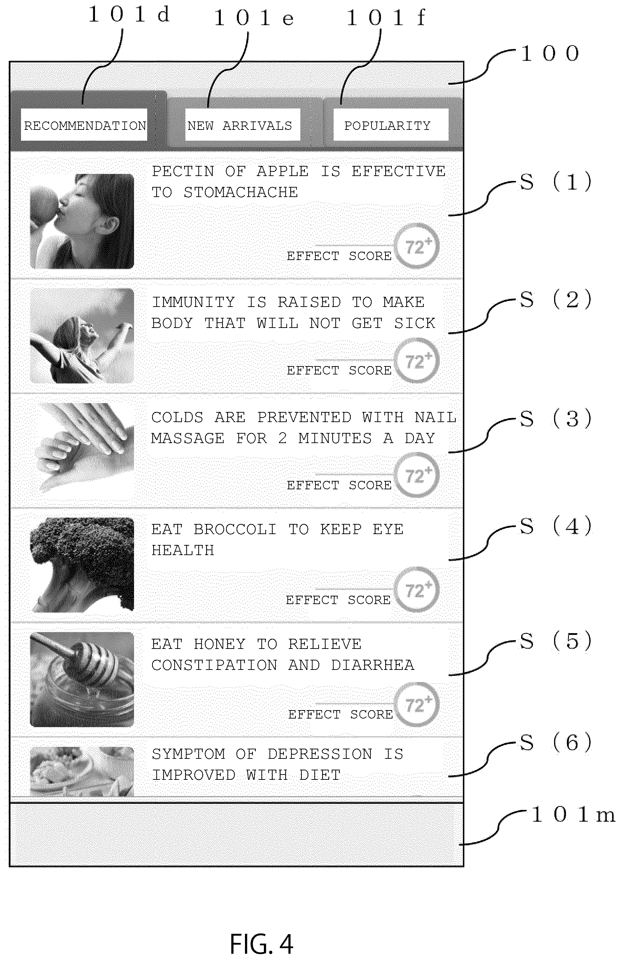

Fig. 4 is a diagram illustrating an example of the article theme list screen displayed on the user terminal 100. In this example, as articles S(i) of the health information, for example, "S(1) pectin of an apple is effective for stomachache", "S(2) immunity is raised to make a body that will not get sick", "S (3) colds are prevented with nail massage for 2 minutes a day" ... and "S(6) symptom of depression is improved with diet", which are created by the administrator of the administrator terminal 300, are displayed on the article theme list screen.

At the top of the article theme list screen, tabs showing article classifications, a recommendation 101d additionally displayed on the article to be recommended for browsing, new arrivals 101e indicating that there are new articles, and a popularity 101f indicating that there is a popular article are displayed. The example of the article theme list screen illustrated in Fig. 4 relates to recommended articles.

For example, when the user selects (taps) a theme of an article that is most interested from the article theme list, the user terminal 100 transmits an instruction to download the selected article S(i) and display the downloaded article S(i) on the display 101, specifically a URL assigned to the article, to the web server 200 (step S6).

Before the web server 200 provides the user with the article S(i) corresponding to the received URL, the web server 200 reads two questions Q1 and Q 2 corresponding to the article S(i) from the database 201 and transmits the two questions Q1 and Q2 to the user terminal 100 so as to present the two questions Q1 and Q2 to the user as questionnaire screens.

Specifically, for example, when it is assumed that the user selects a theme article S (1) "pectin of an apple is effective to stomachache" as a browsing subject, the web server 200 first transmits data of a first questionnaire screen corresponding to the question Q1 to the user terminal 100 as a question corresponding to the article S(1) (step S7).

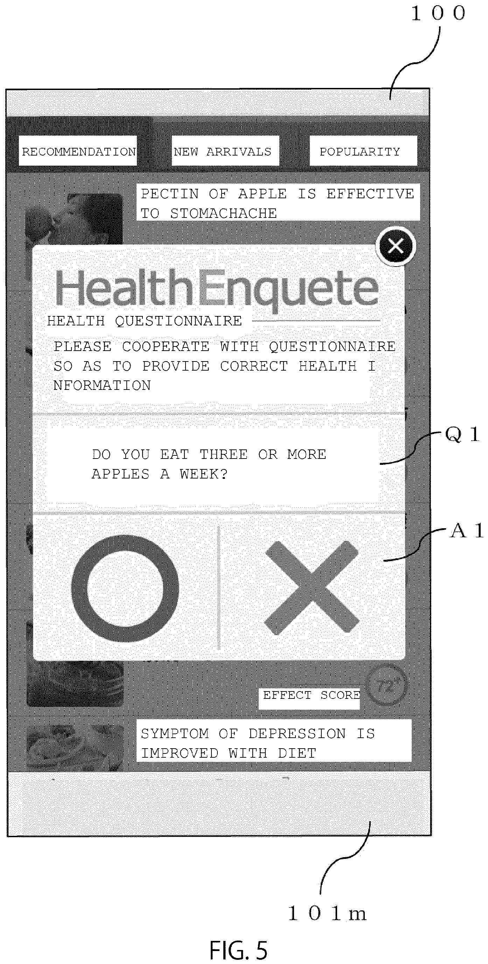

Fig. 5 is a diagram illustrating an example of the first questionnaire screen displayed on the user terminal 100. In this example, on the first questionnaire screen, "Do you eat three or more apples a week?" is displayed as the question Q1, and a selection icon "o" indicating "Yes" and a selection icon "×" indicating "No" are displayed as the answer A1.

When the user selects (taps) the icon "○" or "×", the user terminal 100 transmits the data of the answer A1 to the web server 200 according to the selection (step S8).

The web server 200 temporarily stores the data of the received answer A1 in the controller 202, and then transmits data of a second questionnaire screen corresponding to the next question Q2 to the user terminal 100 (step S9).

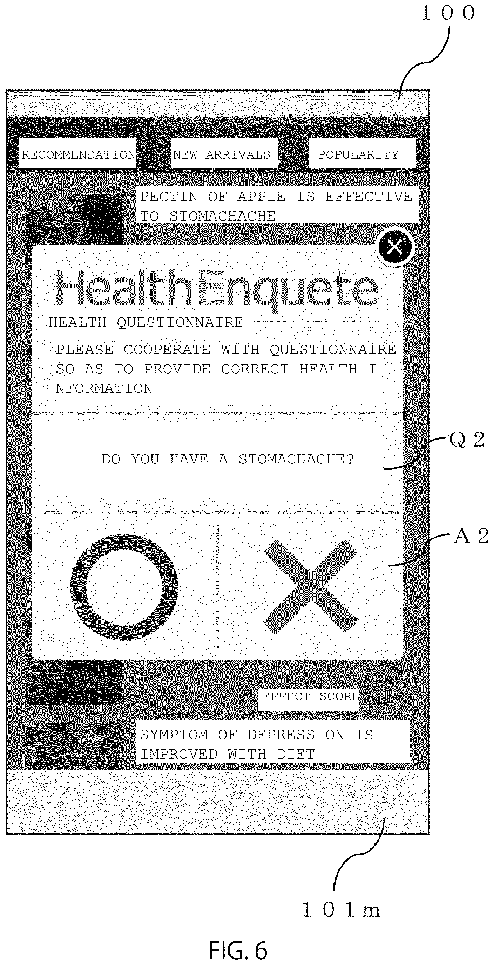

Fig. 6 is a diagram illustrating an example of the second questionnaire screen displayed on the user terminal. In this example, on the second questionnaire screen, "Do you have a stomachache?" is displayed as the question Q2, and a selection icon "○" indicating "Yes" and a selection icon "×" indicating "No" are displayed as the answer A2.

When the user selects (taps) the icon "○" or "×", the user terminal 100 transmits the data of the answer A2 to the web server 200 according to the selection (step S10).

The web server 200 temporarily stores the data of the received answer A2 in the controller 202, and then transmits to the administrator terminal 300 that the data of the answers A1 and A2 have been acquired, as the answer to the questions Q1 and Q2 on the article S (i) (step S11). Further, the web server 200 executes correlation analysis with statistical data based on the article S(i) and the data of the answers A1 and A2 (step S12) .

In the correlation analysis, an effect evaluation (hereinafter referred to as "effect score E") is obtained by using a significant difference determination method by a test. A plurality of types of test are known, and any one of these can be used. However, according to the following consideration results, the use of other tests is not excluded, but it is preferable to use the following z test of a type 1 as the health information utilization system. As a known test, for example, there are type 1: a test (z test) of a difference of population rates in different groups, type 2: a test (chi-square test) of a difference of population rates in the same group, type 3: a test (z test) of a difference of population rates in the item of the same category, and type 4: a test (z test) of a difference of population rates in the whole and partial categories.

Here, which type of the test is most effective is discussed. Although an effect score cannot be directly obtained by a test that only acquires a significant difference, a numerical value of the effect score is found (discussion 1). Since type 3 and type 4 are the significant difference tests for those with dependency on the questionnaire, type 1 and type 2 are rather preferable (discussion 2). The tests are performed on the same case for type 1 and type 2, and a test that gives a more remarkable result on both of them is selected (discussion 3). From the test result of the discussion 3, the z test of the type 1 is determined as most effective (discussion 4).

In the z test of the type 1, the number of respondents of two different groups is set as n1 (first group) and n2 (second group), respectively. It is assumed that, for the item in the first group, the number of affirmative persons is a and the number of negative persons is b. In addition, it is assumed that, in the same item in the second group, the number of affirmative persons is c and the number of negative persons is d. In this way, the following equation is established.

$$n1 = a + b; \quad n2 = c + d$$

In addition, when the answer ratio of the affirmative persons in the first group is p1 and the answer ratio of the affirmative persons in the second group is p2, the following expression is established.

$$p1 = a/n1, \quad p2 = c/n2$$

In the z test in the significant difference determination method, a statistical amount T is expressed by Mathematical Formula 1 below.

[Mathematical Formula 1]

$$T = \frac{p_1 - p_2}{\sqrt{p(1-p)\left(\frac{1}{n_1} + \frac{1}{n_2}\right)}}$$

In Mathematical Formula 1, p is expressed by Mathematical Formula 2 below.

[Mathematical Formula 2]

$$p = \frac{n_1 p_1 + n_2 p_2}{n_1 + n_2}$$

From the z test, a significant point is 1.96 (absolute value; the same applies below) . When an absolute value of the statistical amount T is greater than or equal to the significant point, it can be said that a ratio of the affirmative persons in the sum group of the first group and the second group, that is, the population, has a significant difference between the first group and the second group. In addition, if an advantage point $1.96 \approx 2.0$ is multiplied by 25 times, the effect score E becomes a numerical value of 100. Thus, since it is considered that there is generally familiarity, the effect score E is represented by 25 times the statistical amount T (E = 25T).

In addition, the probability P automatically determined when the statistical amount T is obtained has a correlation such that, when the value of the statistical amount T is small or large, the probability P becomes large or small. Since the value P is troublesome in manual calculation, a function in a spreadsheet program such as Excel (registered trademark) may be used.

That is, for example, when Excel is used, the following function is input to an arbitrary cell and an Enter key is pressed.

= 2*(1 - NORMSDIST (statistical amount T))

The significant probability of the value P is 0.05. When the value P is less than or equal to 0.05, it can be said that a ratio of the affirmative persons in the population of the first group and the second group has a significant difference between the first group and the second group.

Next, as illustrated in Fig. 5 and Fig. 6, in the specific example of the answers A1 and A2 to the questions Q1 and Q2 of the theme of the article S(1) "pectin of an apple is effective in plurality", the statistical amount T and the value P, and the effect score E of the z test are obtained.

In this case, the first group is a group of respondents who "eat an apple" and the second group is a group of respondents who "do not eat an apple. " In addition, the affirmative persons in each group are respondents who "have a stomachache" and the negative persons are respondents who "do not have a stomachache."

In this case, it is assumed that there are 200 respondents who "eat an apple" and 200 respondents who "do not eat an apple" (n1 = n2 = 200). In addition, it is assumed that, among the respondents who "eat an apple", there are 94 respondents (a = 94) who "have a stomachache" and 106 respondents (b = 106) who "do not have a stomachache." On the other hand, it is assumed that, among the respondents who "do not eat an apple", there are 100 respondents who "have a stomachache" and 100 respondents who "do not have a stomachache" (c = d = 100).

In the case of this assumption, the ratio of the respondents who "have a stomachache" in the group of the respondents who "eat an apple" is 0.47 (p1 = 0.47), and the ratio of the respondent who "have a stomachache" in the group of the respondents who "do not eat an apple" is 0.5 (p2 = 0.5). Therefore, the value p according to Mathematical Formula 2 is 0.485.

As a result, the statistical amount T obtained based on Mathematical Formula 1 has the following value.

T = 0.600270182

That is, since the value of the statistical amount T is considerably smaller than the significant point of 1.96, there is no significant difference between the respondents who "eat an apple" and the respondents who "do not eat an apple", and the effect of "pectin of an apple is effective to stomachache" cannot be found. In this case, the effect score E of the z test is about 15 (E = 25T).

In addition, the value P obtained by the function of Excel becomes the following value in the above assumption.

$$P = 0.5483261872593940$$

That is, since the value P is considerably larger than the significant probability of 0.05, there is no significant difference between the respondents who "eat an apple" and the respondents who "do not eat an apple", and the effect of "pectin of an apple is effective to stomachache" cannot be found.

Next, the number of persons in the population is 10 times . That is, it is assumed that there are 2,000 respondents who "eat an apple" and 2,000 respondents who "do not eat an apple" (n1 = n2 = 2,000). In addition, it is assumed that, among the respondents who "eat an apple", there are 940 respondents (a = 940) who have a stomachache and 1,060 respondents (b = 1,060) who "do not have a stomachache . " On the other hand, it is assumed that, among the respondents who "do not eat an apple", there are 1,000 respondents who "have a stomachache" and 1,000 respondents who "do not have a stomachache" (c = d = 1,000).

In the case of this assumption, as in the case where the number of persons in the population is assumed to be 200, the ratio of the respondents who "have a stomachache" in the group of the respondents who "eat an apple" is 0.47 (p1 = 0.47), and the ratio of the respondent who "have a stomachache" in the group of the respondents who "do not eat an apple" is 0.5 (p2 = 0.5) . Therefore, according to Mathematical Formula 2, the value p becomes 0.485 as in the case where the number of persons in the population is 200.

It should be noted that the statistical amount T obtained based on Mathematical Formula 1 has the following value.

$$T = 1.898220988$$

That is, in the case of this assumption, since the value of the statistical amount T is smaller than the significant point of 1.96, there is no significant difference between the respondents who "eat an apple" and the respondents who "do not eat an apple", and the effect of "pectin of an apple is effective to stomachache" cannot be found. In this case, the effect score E of the z test is about 47 (E = 25T).

In addition, the value P obtained by the function of Excel becomes the following value in the above assumption.

$$P = 0.0576669771862157$$

In this case, since the value P is larger than the significant probability of 0.05, there is no significant difference between the respondents who "eat an apple" and the respondents who "do not eat an apple", and the effect of "pectin of an apple is effective to stomachache" cannot be found.

Next, the number of persons in the population is 100 times . That is, it is assumed that there are 20,000 respondents who "eat an apple" and 20,000 respondents who "do not eat an apple" (n1 = n2 = 20,000). In addition, it is assumed that, among the respondents who "eat an apple", there are 9,400 respondents (a = 9,400) who have a stomachache and 10, 600 respondents (b = 10, 600) who "do not have a stomachache . " On the other hand, it is assumed that, among the respondents who "do not eat an apple", there are 10,000 respondents who "have a stomachache" and 10,000 respondents who "do not have a stomachache" (c = d = 10,000).

In the case of this assumption, as in the case where the number of persons in the population is assumed to be 200, the ratio of the respondents who "have a stomachache" in the group of the respondents who "eat an apple" is 0.47 (p1 = 0.47), and the ratio of the respondent who "have a stomachache" in the group of the respondents who "do not eat an apple" is 0.5 (p2 = 0.5) . Therefore, according to Mathematical Formula 2, the value p becomes 0.485 as in the case where the number of persons in the population is 200.

It should be noted that the statistical amount T obtained based on Mathematical Formula 1 has the following value.

$$T = 6.002701824$$

That is, in the case of this assumption, since the value of the statistical amount T is significantly larger than the significant point of 1.96, there is a significant difference between the respondents who "eat an apple" and the respondents who "do not eat an apple", and the great effect of "pectin of an apple is effective to stomachache" can be found. In this case, the effect score E of the z test is about 150 (E = 25T) .

In addition, the value P obtained by the function of Excel becomes the following value in the above assumption.

$$P = 0.0000000019406081$$

In this case, since the value P is significantly less than the significant probability of 0.05, there is a significant difference between the respondents who "eat an apple" and the respondents who "do not eat an apple", and the great effect of "pectin of an apple is effective to stomachache" can be found.

Therefore, as the number of persons in the population increases, a significant difference between the respondents who "eat an apple" and the respondents who "do not eat an apple" increases, and the effect of "pectin of an apple is effective to stomachache" is also improved. Therefore, as the number of the users of the user terminals 100 increases, the effect score E of the z test increases. When the effect score E is a predetermined value, for example, 72 or more, the health information utilization system determines that it has a significant difference, and thus only highly reliable information that would be correct can be provided to the user. Accordingly, if necessary, if the number of persons in the population is small, it is also lawful to provide the user with the result calculated by multiplying each answer number by 100.

In the sequence diagram of Fig. 2, after the web server 200 performs the correlation analysis in step S12, the web server 200 transmits data of the comment screen to the user terminal 100 (step S13). It should be noted that the execution of step S13 is not performed whenever the user transmits A1 and A2, but may be performed periodically or irregularly, for example, once a day, once a week, or the like.

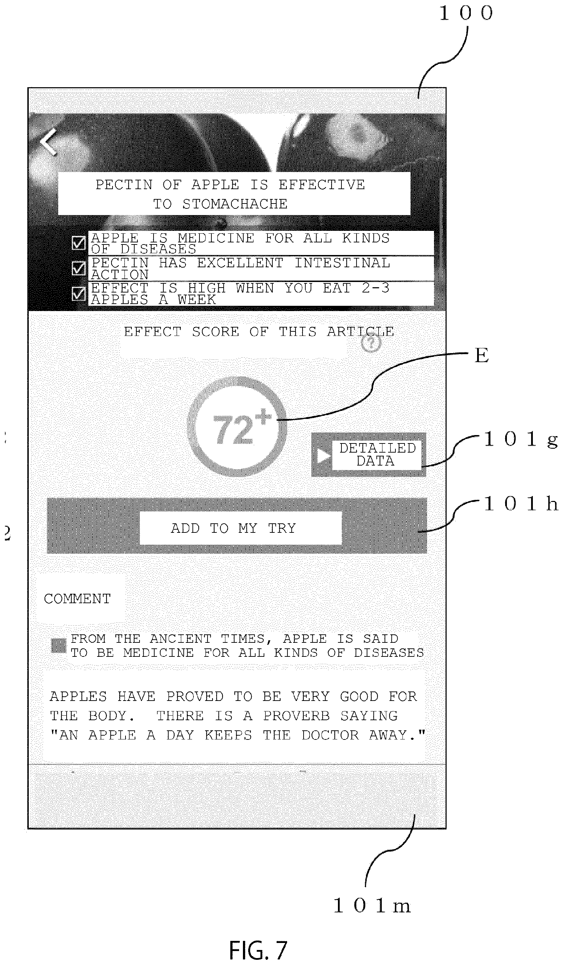

Fig. 7 is a diagram illustrating an example of the comment screen displayed on the user terminal 100. In this example, the effect score E of the z test of the article "pectin of an apple is effective to stomachache" is a positive value of 72. Since the value of the statistical amount T in this case is about 2.88 and the value of the statistical amount T is larger than the significant point of 1.96, there is a significant difference between the respondents who "eat an apple" and the respondents who "do not eat an apple", and there is the effect of "pectin of an apple is effective to stomachache." In the comment, the article of the effect is shown.

In the comment screen example of Fig. 7, a tab 101g of "detailed data" in the effect score E of the detailed z-test and the tab 101h of "add to my try" showing a theme for challenging for health promotion are displayed. When the user selects (taps) the tab of "detailed data" or "addition of my try", the user terminal 100 transmits the selected instruction to the web server 200 (step S14).

The web server 200 determines whether the instruction received from the user terminal 100 is a request for detailed data or a request for addition of my try (step S15). As a result of the execution of step S15, when the web server 200 determines that the instruction from the user terminal 100 is the request for detailed data (step S15; YES), the web server 200 transmits data of an effect score detailed screen to the user terminal 100 (step S16).

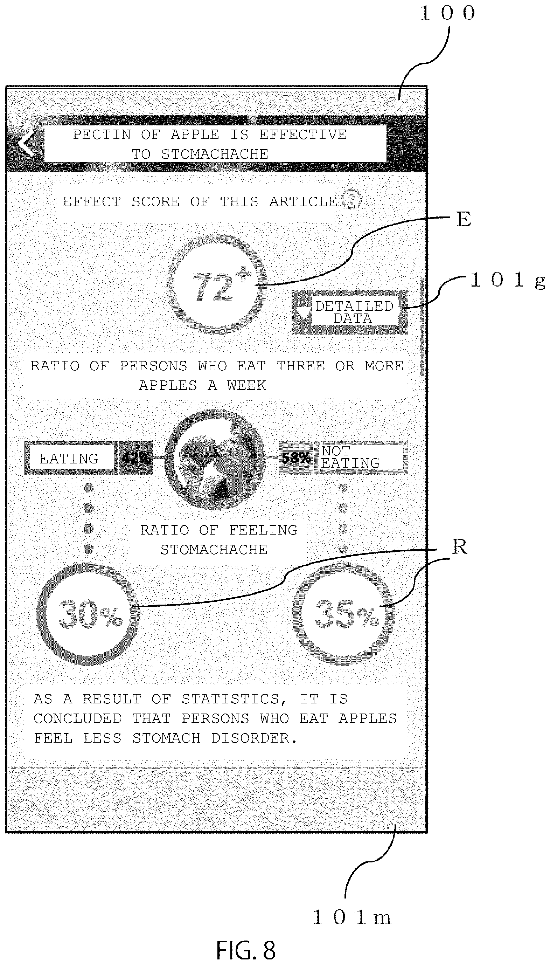

Fig. 8 is a diagram illustrating an example of the effect score detailed screen displayed on the user terminal 100. The effect score E, the tab 101g of "detailed data", the ratio R of feeling stomachache of the person who eats an apple and a person who does not eat an apple, and the comment thereof are displayed on the screen.

When the tab 101g of "detailed data" in Fig. 8 is tapped, a further detailed explanation, a reference book referred to when creating an article, a purchasing method thereof, and the like are displayed.

On the other hand, as a result of the execution of step S15, when the web server 200 determines that the instruction from the user terminal 100 is the request for addition of my try (step S15; NO), the web server 200 transmits the data of the my try addition screen to the user terminal 100 (step S17) .

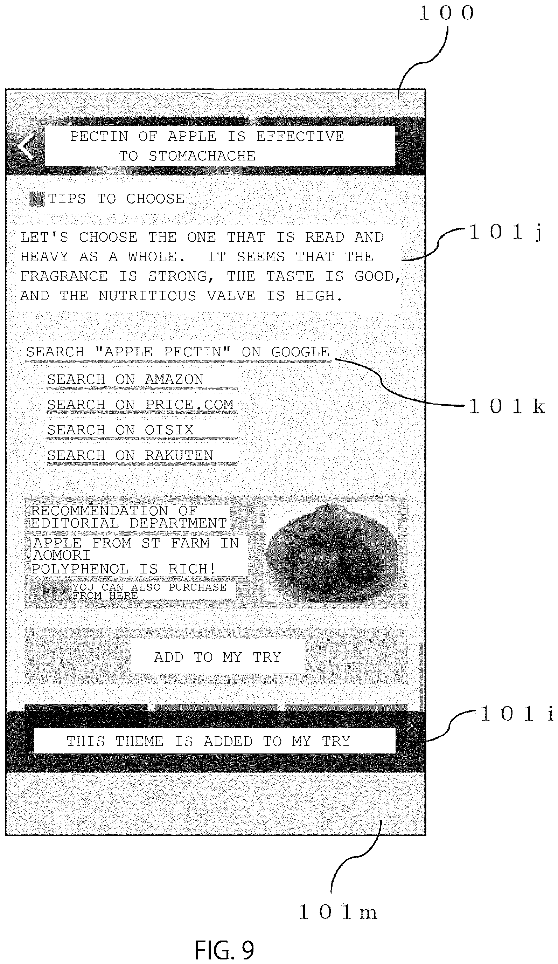

Fig. 9 is a diagram illustrating an example of the my try addition screen displayed on the user terminal 100. A message 101i indicating that a theme of challenging the habit of continuously eating an apple is newly added, an advice 101j to be noted in eating an apple, a further detailed information search tab 101k, and the like are displayed on the screen. After that, the web server 200 transmits, to the user terminal 100, data of a my try management screen including the theme newly added by the user (step S18).

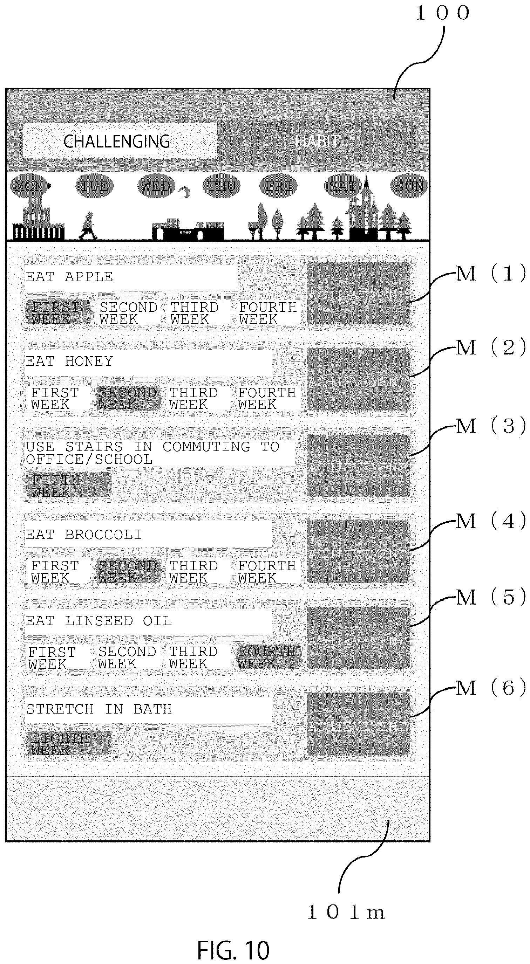

Fig. 10 is a diagram illustrating an example of the my try management screen displayed on the user terminal 100. For the purpose of health promotion, the theme newly added by the user is displayed on the screen together with the theme that the user has added in the past.

That is, in this example, my try of "eating an apple" is additionally displayed together with my try of "eating honey" . Then, achievements M(1), M(2), M(3), and the like, which are tabs for self-checking an achievement situation, for example, every week, are displayed for each my try. When the user achieves my try of "eating an apple" in the first week, the user may execute a use method such as tapping achievement M(1).

At n (n = 1, 2, 3 ...) weeks, when the user taps achievement M(j) of any one my try, the user terminal 100 transmits an instruction of turning on achievement M(j) to the web server 200 (step S19).

When the instruction of turning on achievement M(j), which is received from the user terminal 100, is received, the web server 200 counts the instruction. Then, it is determined whether the number of times of receptions of turning on achievement M(j) has continuously reached predetermined N times (step S20). For example, when the value of N is "4", it means that the user has successfully achieved my try in a period of 4 weeks.

When the web server 200 determines that the number of times of receptions of turning on achievement M(j) has reached N (step S20; YES), the web server 200 determines that my try has become a habit and can transmit, to the user terminal 100, data of a screen that deletes (or invalidates) the achievement M(j) tab (step S21). In this way, it is possible for the user to skip the tap operation weekly for the theme of my try that has become a habit.

After step S21 or when it is determined that the number of times of receptions of turning on achievement M(j) has not reached N (step S20; NO), the web server 200 skips step S21 and determines whether a certain period (for example, one week) has elapsed (step S22).

On the other hand, when it is determined that a certain period has elapsed (step S22; YES), the web server 200 transmits data of a health level screen to the user terminal 100 (step S23) .

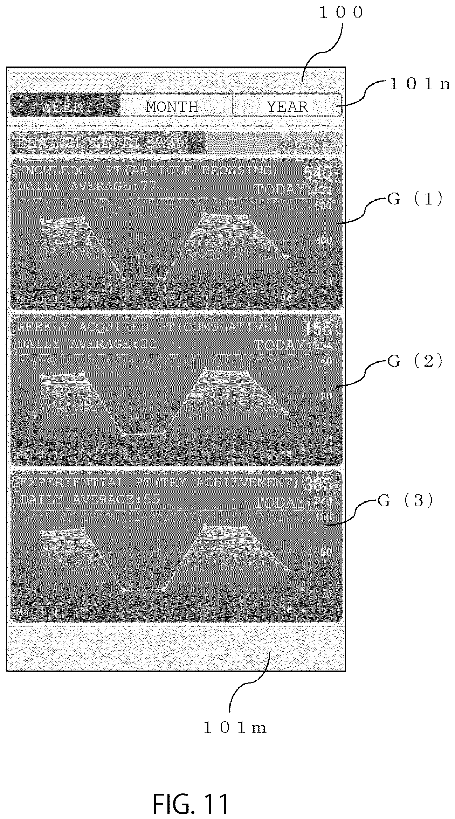

Fig. 11 illustrates the health level screen displayed on the user terminal 100. Cumulatively acquired points of the effect score E in the past week and a transition graph G(1), knowledge points of the article browsing and a transition graph G(2), and experiential points of achievement of my try and a transition graph G (3) are displayed on the screen. In the present embodiment, the health level screen in the past month or the past year can be displayed by tapping the tab 101n on the screen.

After step S23 is executed or when it is determined that a certain period has not elapsed (step S22; NO), the administrator terminal 300 performs a data update process (step S24), and the web server 200 receives the article S(i) and the effect score E that are change data from the administrator terminal 300 (step S25), and updates the data of the database 201.

Optionally, when any one of menu icons 101m of the user terminal 100 is selected (tapped) by the user's operation, the web server 200 transmits data of a screen corresponding to the selected icon to the user terminal 100.

The menu icons 101m include "article", "my try", "health level", and the like. When each of the icons is selected, the article list screen illustrated in Fig. 4, the my try management screen illustrated in Fig. 10, the health level screen illustrated in Fig. 11, and the like are displayed according to the selection result.

In the above-described embodiment, the web server 200 is configured to present two questions corresponding to the article to the user prior to providing the article stored in the database 201 to the user. However, in the modification of the embodiment, three or more questions may be presented to the user. In this case, more reliable health information can be provided to the user.

That is, according to the above-described embodiment, the database 201 of the web server 200 stores a plurality of articles on health information, a plurality of questions on each article, and statistical data of the answer results to the questions.

The web server 200 and the user terminal 100 constitute a presenting means for presenting a plurality of questions corresponding to the article to the user prior to providing the article stored in the database 201 to the user.

In addition, the web server 200 and the user terminal 100 constitute an acquiring means for acquiring each answer result from the user for the plurality of presented questions.

Further, when each answer result is acquired, the web server 200 and the user terminal 100 constitute a providing means for reading the statistical data corresponding to each answer result from the database 201 and providing the read statistical data to the user.

On the other hand, the administrator terminal 300 constitutes an updating means for updating the statistical data based on the answer result obtained by the web server 200.

In addition, according to the embodiment, the health information utilization system configured by the user terminal 100, the web server 200, and the administrator terminal 300 realizes the health information utilization method including:

a first step of providing the plurality of questions corresponding to the article to the user by using the network 400 prior to providing the user with the articles stored in the database 201 in which the plurality of articles on the health information, the plurality of questions on each article, and the statistical data of the answer results to the questions are stored;

a second step of acquiring each answer result from the user with respect to the plurality of questions presented in the first step;

a third step of reading the statistical data corresponding to each answer result from the database 201 when each answer result is acquired in the second step and providing the read statistical data to the user; and

a fourth step of updating the statistical data based on the answer result acquired in the second step.

Furthermore, according to the above-described embodiment, the user terminal 100 can install or download the health information utilization program via the external memory or the network 400.

That is, the health information utilization program causes the user terminal 100 to execute:

a first step of accessing the web server 200 in which the plurality of articles on health information, the plurality of questions on each article, and the statistical data of the answer results to the questions are stored;

a second step of presenting the plurality of questions corresponding to the article to the user prior to providing the article stored in the web server 200 to the user;

a third step of transmitting, to the web server 200, each answer result from the user with respect to the plurality of questions presented in the second step; and

a fourth step of receiving, from the web server 200, the statistical data corresponding to each answer result transmitted in the third step, and providing the received statistical data to the user.

In the above-described embodiment, the user terminal 100 is configured by a smartphone having a phone function and a communication function, but can also be configured by a notebook personal computer, a desktop personal computer, a digital portable terminal, or the like, which has only a communication function.

In addition, in the above-described embodiment, the administrator terminal 300 is a notebook personal computer, but can also be a desktop personal computer, a digital portable terminal, or the like.

Furthermore, in the above-described embodiment, the web server 200 and the administrator terminal 300 are separately configured, but can be configured by a web server integrated by making the web server 200 have the function of the administrator terminal 300.

**Claims**

1. A health information utilization system comprising:

   a providing means for providing a task relating to health information to a user;
   a receiving means for receiving information indicating that the task provided by the providing means has been achieved; and
   a stopping means for stopping receiving the information when the number of times of receptions of the information by the receiving means exceeds a predetermined number.

2. The health information utilization system according to claim 1, comprising a resuming means for, after the reception is stopped by the stopping means, resuming provision of a stopped task when a stop release instruction is transmitted from the user.

3. The health information utilization system according to claim 1 or 2, further comprising:

   a database in which a plurality of articles on health information, a plurality of questions on each article, and statistical data of answer results to the questions are stored;
   a presenting means for presenting the plurality of questions corresponding to the article to the user prior to providing the article stored in the database to the user;
   an acquiring means for acquiring each answer result from the user with respect to the plurality of questions presented by the presenting means;
   a providing means for reading the statistical data corresponding to each answer result from the database when each answer result is acquired by the acquiring means and providing the read statistical data to the user; and
   an updating means for updating the statistical data based on the answer result acquired by the acquiring means, wherein the providing means provides the user with a task relating to health information based on the answer result acquired by the acquiring means.

4. A health information utilization program for causing a user terminal to execute:

   a step of providing a task relating to health information to a user;
   a step of receiving information indicating that the task provided by the providing means has been achieved; and
   a step of stopping receiving the information when the number of times of receptions of the information by the receiving means exceeds a predetermined number.

FIG. 1

FIG. 2

100

101a

**Health Try**

READ, KNOW, AND PARTICIPATE.
EVERYONE, BE HEALTHY.

101b

101c

START FROM THE BEGINNING

FIG. 3

101d    101e    101f

100

| RECOMMENDATION | NEW ARRIVALS | POPULARITY |

PECTIN OF APPLE IS EFFECTIVE TO STOMACHACHE

EFFECT SCORE  72⁺

S(1)

IMMUNITY IS RAISED TO MAKE BODY THAT WILL NOT GET SICK

EFFECT SCORE  72⁺

S(2)

COLDS ARE PREVENTED WITH NAIL MASSAGE FOR 2 MINUTES A DAY

EFFECT SCORE  72⁺

S(3)

EAT BROCCOLI TO KEEP EYE HEALTH

EFFECT SCORE  72⁺

S(4)

EAT HONEY TO RELIEVE CONSTIPATION AND DIARRHEA

EFFECT SCORE  72⁺

S(5)

SYMPTOM OF DEPRESSION IS IMPROVED WITH DIET

S(6)

101m

FIG. 4

100

RECOMMENDATION    NEW ARRIVALS    POPULARITY

PECTIN OF APPLE IS EFFECTIVE
TO STOMACHACHE

# HealthEnquete

HEALTH QUESTIONNAIRE

PLEASE COOPERATE WITH QUESTIONNAIRE
SO AS TO PROVIDE CORRECT HEALTH I
NFORMATION

DO YOU EAT THREE OR MORE
APPLES A WEEK?

Q1

A1

O    X

EFFECT SCORE    72⁺

SYMPTOM OF DEPRESSION IS
IMPROVED WITH DIET

101m

FIG. 5

1 0 0

RECOMMENDATION    NEW ARRIVALS    POPULARITY

PECTIN OF APPLE IS EFFECTIVE
TO STOMACHACHE

# HealthEnquete

HEALTH QUESTIONNAIRE

PLEASE COOPERATE WITH QUESTIONNAIRE
SO AS TO PROVIDE CORRECT HEALTH I
NFORMATION

DO YOU HAVE A STOMACHACHE?    Q 2

A 2

EFFECT SCORE  72°

SYMPTOM OF DEPRESSION IS
IMPROVED WITH DIET

1 0 1 m

FIG. 6

100

PECTIN OF APPLE IS EFFECTIVE
TO STOMACHACHE

☑ APPLE IS MEDICINE FOR ALL KINDS
OF DISEASES
☑ PECTIN HAS EXCELLENT INTESTINAL
ACTION
☑ EFFECT IS HIGH WHEN YOU EAT 2-3
APPLES A WEEK

EFFECT SCORE OF THIS ARTICLE ⑦

E

72⁺

▶ DETAILED
DATA
101g

ADD TO MY TRY
101h

COMMENT

■ FROM THE ANCIENT TIMES, APPLE IS SAID
TO BE MEDICINE FOR ALL KINDS OF DISEASES

APPLES HAVE PROVED TO BE VERY GOOD FOR
THE BODY.  THERE IS A PROVERB SAYING
"AN APPLE A DAY KEEPS THE DOCTOR AWAY."

101m

FIG. 7

100

PECTIN OF APPLE IS EFFECTIVE
TO STOMACHACHE

EFFECT SCORE OF THIS ARTICLE ?

E

72⁺

101g

▼ DETAILED
DATA

RATIO OF PERSONS WHO EAT THREE OR MORE
APPLES A WEEK

EATING  42%    58% NOT
                   EATING

RATIO OF FEELING
STOMACHACHE

R

30%          35%

AS A RESULT OF STATISTICS, IT IS
CONCLUDED THAT PERSONS WHO EAT APPLES
FEEL LESS STOMACH DISORDER.

101m

FIG. 8

18

100

PECTIN OF APPLE IS EFFECTIVE
TO STOMACHACHE

TIPS TO CHOOSE

LET'S CHOOSE THE ONE THAT IS READ AND
HEAVY AS A WHOLE.  IT SEEMS THAT THE
FRAGRANCE IS STRONG, THE TASTE IS GOOD,
AND THE NUTRITIOUS VALVE IS HIGH.

101j

SEARCH "APPLE PECTIN" ON GOOGLE

SEARCH ON AMAZON

SEARCH ON PRICE.COM

SEARCH ON OISIX

SEARCH ON RAKUTEN

101k

RECOMMENDATION OF
EDITORIAL DEPARTMENT
APPLE FROM ST FARM IN
AOMORI
POLYPHENOL IS RICH!

▶▶▶ YOU CAN ALSO PURCHASE
FROM HERE

ADD TO MY TRY

THIS THEME IS ADDED TO MY TRY

101i

101m

FIG. 9

100

| CHALLENGING | HABIT |

MON  TUE  WED  THU  FRI  SAT  SUN

EAT APPLE

FIRST WEEK   SECOND WEEK   THIRD WEEK   FOURTH WEEK

ACHIEVEMENT

M (1)

EAT HONEY

FIRST WEEK   SECOND WEEK   THIRD WEEK   FOURTH WEEK

ACHIEVEMENT

M (2)

USE STAIRS IN COMMUTING TO OFFICE/SCHOOL

FIFTH WEEK

ACHIEVEMENT

M (3)

EAT BROCCOLI

FIRST WEEK   SECOND WEEK   THIRD WEEK   FOURTH WEEK

ACHIEVEMENT

M (4)

EAT LINSEED OIL

FIRST WEEK   SECOND WEEK   THIRD WEEK   FOURTH WEEK

ACHIEVEMENT

M (5)

STRETCH IN BATH

EIGHTH WEEK

ACHIEVEMENT

M (6)

101m

FIG. 10

EP 3 333 800 A1

100

101n

WEEK      MONTH      YEAR

HEALTH LEVEL:999          1,200/2,000

KNOWLEDGE PT(ARTICLE BROWSING)  540
DAILY AVERAGE:77          TODAY 13.33

600

300

0

March 12  13  14  15  16  17  18

G（1）

WEEKLY ACQUIRED PT(CUMULATIVE)  155
DAILY AVERAGE:22          TODAY 10:54

40

20

0

March 12  13  14  15  16  17  18

G（2）

EXPERIENTIAL PT(TRY ACHIEVEMENT) 385
DAILY AVERAGE:55          TODAY 17:40

100

50

0

March 12  13  14  15  16  17  18

G（3）

101m

FIG. 11

21

## INTERNATIONAL SEARCH REPORT

| | International application No. |
|---|---|
| | PCT/JP2016/070040 |

A. CLASSIFICATION OF SUBJECT MATTER
*G06Q50/22*(2012.01)i

According to International Patent Classification (IPC) or to both national classification and IPC

B. FIELDS SEARCHED

Minimum documentation searched (classification system followed by classification symbols)
G06Q50/22

Documentation searched other than minimum documentation to the extent that such documents are included in the fields searched
Jitsuyo Shinan Koho 1922–1996 Jitsuyo Shinan Toroku Koho 1996–2016
Kokai Jitsuyo Shinan Koho 1971–2016 Toroku Jitsuyo Shinan Koho 1994–2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2000-37354 A (Omron Corp.), 08 February 2000 (08.02.2000), entire text; all drawings (Family: none) | 1-4 |
| A | JP 2015-65590 A (Sharp Corp.), 09 April 2015 (09.04.2015), particularly, paragraphs [0119] to [0138] & US 2015/0088287 A1 particularly, paragraphs [0144] to [0165] & CN 104460573 A | 1-4 |
| A | JP 2008-310410 A (Yugen Kaisha Awake), 25 December 2008 (25.12.2008), entire text; all drawings (Family: none) | 1-4 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 14 September 2016 (14.09.16) | Date of mailing of the international search report 27 September 2016 (27.09.16) |
|---|---|
| Name and mailing address of the ISA/ Japan Patent Office 3-4-3,Kasumigaseki,Chiyoda-ku, Tokyo 100-8915,Japan | Authorized officer<br><br>Telephone No. |

Form PCT/ISA/210 (second sheet) (January 2015)

## INTERNATIONAL SEARCH REPORT

| International application No. |
|---|
| PCT/JP2016/070040 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2012-230503 A  (Nippon Telegraph and Telephone Corp.), 22 November 2012 (22.11.2012), entire text; all drawings (Family: none) | 1-4 |
| A | JP 2015-26208 A  (Takafumi KASHIWA), 05 February 2015 (05.02.2015), entire text; all drawings (Family: none) | 1-4 |

Form PCT/ISA/210 (continuation of second sheet) (January 2015)

**EP 3 333 800 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008181188 A